# DEMANDE DE BREVET EUROPEEN

(11) **EP 1 500 914 A1**
(43) Date de publication de la demande: **26.01.2005**
(21) Numéro de dépôt: 04291548.8
(22) Date de dépôt: 18.06.2004
(51) Int. Cl.: G01J 1/42, G01J 1/50, A42B 1/24, A61F 9/04, A42B 1/06

(54) **Dispositif destiné à prévenir les risques de surexposition aux rayonnements néfastes du soleil**

(30) Priorité: 23.07.2003 FR 0308998
(71) Demandeur: L'OREAL, 75008 Paris (FR)
(72) Inventeur: Fontaine, Michel, 75008 Paris (FR)
(74) Mandataire: Schmit, Charlotte

(57) **Abrégé**

La présente demande concerne un dispositif (1) destiné à prévenir les risques de surexposition aux rayonnements néfastes du soleil comprenant : i) des moyens de détection, comprenant au moins un capteur sensible à un rayonnement solaire, notamment UV ; ii) des moyens couplés aux moyens de détection pour générer un signal représentatif de la quantité de rayonnement reçue par les moyens de détection, et iii) des moyens (2) pour la fixation du dispositif (1) sur la tête d'un sujet, de sorte que le(s) capteur(s) soi(en)t orienté(s) de façon sensiblement perpendiculaire à la verticale du sujet lorsque celui-ci est actif au soleil, le dispositif étant configuré sous forme d'une casquette, les moyens de détection (12) étant disposés sur une première visière (4), destinée à être positionnée en regard de la nuque de l'individu, la casquette comprend une seconde visière (3), située à l'opposé de la première.

## Description

La présente invention a trait à un dispositif pour la détection d'un rayonnement solaire, notamment ultraviolet ou infrarouge, en vue de prévenir les risques de surexposition aux rayonnements néfastes du soleil, en particulier pour les enfants.

Il a été prouvé cliniquement que l'exposition du corps humain au rayonnement solaire augmente de manière sensible les risques de développer un cancer de la peau. La prévention du cancer de la peau a été jusqu'a présent assurée par l'utilisation de produits à base de filtres solaires, permettant une exposition prolongée au soleil.

En dépit de leur efficacité reconnue à protéger la peau contre les rayonnements néfastes du soleil, ces produits montrent une efficacité limitée dans le temps, efficacité encore réduite par la transpiration, ou par l'exercice de certaines activités physiques, en particulier, la natation.

Indépendamment de l'activité physique exercée ou non par le sujet, l'activité des produits de protection contre les effets du soleil se dégrade plus ou moins rapidement sous la simple action du rayonnement UV.

Il en résulte que ces produits doivent être appliqués régulièrement, selon une fréquence variable en fonction de l'activité exercée par l'individu concerné, de son type de peau, ou du degré d'ensoleillement.

A l'expérience, il s'avère que bon nombre de sujets oublient purement et simplement d'appliquer à nouveau du produit. Dans d'autres cas, le sujet a du mal à évaluer la fréquence d'application en fonction de l'heure de la journée ou en fonction des jours, dépendant en particulier du degré d'ensoleillement. La fréquence d'application de ces produits varie en outre en fonction du type de peau.

Il existe donc un besoin pour un système permettant, de façon simple et efficace, de renseigner les sujets exposés au soleil, sur le moment où le produit de protection doit être appliqué à nouveau, ou sur le moment où l'exposition au soleil doit être arrêtée.

Il existe des dispositifs permettant de mesurer une quantité de rayonnement solaire reçue. De tels dispositifs sont soit sous forme d'unités plus ou moins volumineuses, présentes par exemple sur les plages, à la disposition de tout le monde, ou que le sujet peut avoir avec lui, notamment sous forme d'un système qu'il porte à la manière d'une montre. Alternativement, le dispositif peut être intégré au packaging d'un produit de protection contre les effets du soleil.

De tels dispositifs sont décrits notamment dans les brevets US 5 986 273, US 5 378 896, US 4 535 244, US 3 710 115, US 4 863 282, ou WO 91/04469. Ces systèmes utilisent soit des photocellules, soit des compositions photochromiques ou thermochromiques, disposées dans une ou plusieurs zones qui, change(nt) de couleur en fonction de la quantité de radiation reçue.

Un problème majeur propre à tous ces systèmes tient au fait que l'information qu'ils donnent, quant à la quantité de radiation reçue, ne correspond pas souvent à la quantité effectivement reçue par un individu se trouvant à une distance plus ou moins grande du système de détection, et exposé de manière souvent très différente de l'exposition à laquelle est soumise le capteur du dispositif.

Il en est de même pour les systèmes que l'on porte à la manière d'une montre, et qui, du fait de leur orientation moyenne relativement aux rayons du soleil, donnent une information généralement bien en dessous de la réalité.

On connaît en particulier du document US-6,132,681 un indicateur permettant de mesurer une quantité de rayonnement UV reçu, cet indicateur pouvant être attaché à un vêtement.

Un problème qui apparaît même avec ce type d'indicateur tient au fait qu'il est disposé sur une partie du vêtement qui ne correspond pas nécessairement à la zone du corps qui va être la plus intensément soumise à un rayonnement solaire, et de fait, l'information donnée par cet applicateur est bien en dessous de la réalité.

Le danger est alors grand pour le sujet qui, au final, est insuffisamment protégé, car prévenu trop tardivement de la nécessité de se protéger plus efficacement, par exemple par l'application d'une nouvelle couche de crème de protection solaire, ou tout simplement en se mettant à l'ombre. Le danger est encore plus grand pour les enfants.

Aussi, est-ce un des objets de l'invention que de réaliser un dispositif visant à réduire les risques liés à la surexposition aux rayonnements néfastes du soleil, qui résolve en tout ou partie les problèmes discutés ci-avant en référence aux dispositifs de détection connus.

C'est en particulier un objet de l'invention que de fournir un tel dispositif intégrant des moyens de détection dont l'orientation moyenne relativement au rayonnement solaire est telle, qu'il délivre à celui ou à celle qui le porte, une information plus fiable, permettant de réduire de manière sensible les risques d'une surexposition.

C'est encore un autre objet de l'invention que de fournir un tel dispositif qui soit simple, efficace, et qui ne constitue pas une contrainte sensible pour celui ou celle qui le porte.

D'autres objets encore apparaîtront dans la description détaillée qui suit.

Selon l'invention, ces objets sont atteints en réalisant un dispositif pour réduire les risques liés à la surexposition aux rayonnements néfastes du soleil, comprenant :
i) des moyens de détection, comprenant au moins un capteur sensible à au moins un rayonnement solaire, notamment UV ;
ii) des moyens couplés aux moyens de détection pour générer un signal représentatif de la quantité de rayonnement reçue par les moyens de détection, et
iii) des moyens de fixation du dispositif sur la tête d'un sujet de sorte que le(s) capteur(s) puisse(nt) être orienté(s) de façon sensiblement perpendiculaire à la verticale de l'individu lorsque celui-ci est debout,
le dispositif étant configuré sous forme d'une casquette, les moyens de détection (12) étant disposés sur une première visière (4), destinée à être positionnée en regard de la nuque de l'individu, la casquette comprend une seconde visière (3), située à l'opposé de la première.

De manière surprenante, la demanderesse a en effet constaté que, ainsi disposés, les moyens de détection, étaient dans bon nombre de positions du sujet, en plus de la position debout, exposés de manière sensiblement perpendiculaire au rayonnement solaire. C'est le cas notamment lorsque le sujet est assis, à genoux ou en position accroupie, qui sont des positions fréquentes chez les enfants, notamment quand ils jouent sur la plage.

De ce fait, le risque pour que la quantité de radiation reçue par les capteurs soit inférieure à la quantité de rayonnement à laquelle le sujet est effectivement exposée, et donc de ce fait le risque de surexposition du sujet, s'en trouvent diminués de manière sensible.

Le signal généré en réponse à l'exposition du capteur peut être visuel et/ou sonore.

Les moyens pour générer ledit signal peuvent être distincts des moyens de détection.

A titre d'exemple, les moyens de détection peuvent être constitués d'une photocellule, notamment d'un élément photovoltaïque, qui transforme le rayonnement solaire en un courant électrique.

Ce dernier chauffe une résistance associée ou formant elle même un système thermochrome à une ou plusieurs zones d'un dispositif d'affichage, qui changent de couleur successivement en fonction de la température.

Les zones photochromes du dispositif d'affichage peuvent être disposées sous forme d'une rangée.

A titre purement illustratif, les premières zones de la rangée contiennent un composé qui devient vert lorsqu'une température T0 est atteinte. Les suivantes contiennent un autre composé thermochrome qui devient orange lorsqu'une température T1 >T0 est atteinte. La dernière zone de la rangée contient encore un autre composé thermochrome qui devient rouge lorsqu'une température T2>T1 est atteinte. Le passage au rouge de la dernière zone indique au sujet et/ou à son entourage proche qu'il est temps de réappliquer du produit de protection.

Avantageusement, le passage au rouge de la dernière zone du dispositif d'affichage peut s'accompagner de l'émission d'un signal sonore audible par le sujet et/ou par son proche entourage.

Le signal sonore, notamment sous forme d'une succession de "bips" peut être bien évidemment utilisé de manière indépendante de tout signal visuel.

Selon un mode de réalisation plus sophistiqué, le système peut être associé à des moyens permettant d'entrer le facteur de protection solaire (SPF) d'un produit solaire qui, le cas échéant, a été utilisé par le sujet.

Le SPF, en plus de la quantité de rayonnement reçue, est pris en compte par un calculateur pour la détermination de l'instant où une quantité suffisante de radiation solaire a été reçue par le sujet, l'application d'un produit solaire autorisant une exposition prolongée relativement à l'exposition autorisée en l'absence d'un produit de protection. De même, l'application d'un produit de haut SPF peut, en fonction des circonstances, autoriser une durée d'exposition supérieure à celle permise si le produit appliqué est de faible SPF.

Selon un autre mode de réalisation, ce sont les moyens de détection eux mêmes qui génèrent le signal représentatif de la quantité de rayonnement reçue. Dans ce cas de figure, le signal généré sera de préférence un signal visuel.

Ainsi, le système de détection peut comprendre une ou plusieurs zones incorporant des encres ou colorants photochromes qui changent de couleur de façon réversible en réponse à une exposition à un rayonnement ultraviolet. Typiquement, de telles encres peuvent être activées par un rayonnement UV dont la longueur d'onde varie par exemple de 300 à 360 nm.

Différents pigments et différents types d'encres photochromes peuvent être utilisés et combinés de manière à réagir et à changer de couleur après une certaine durée, ou un certain niveau d'exposition à un rayonnement UV. A titre d'exemple, on peut citer une encre photochrome vendue sous la référence commerciale PHOTOSOL© par PPG. Industries Inc. L'encre photochrome peut être dispersée dans une structure polymérique. A titre d'exemple, peut être cité le produit commercialisé sous la marque PLASTISOL© par la société CIT Industries.

Alternativement, les compositions utilisées dans la (ou les) zone(s) de détection contiennent des composés fluorescents, capables d'absorber un rayonnement UV d'une certaine longueur d'onde et de re-émettre dans une longueur d'onde différente.

Alternativement encore, la (ou les) zone(s) de détection comprend des cristaux liquides thermochromes réagissant à la température. La cellule de détection/affichage peut comprendre une pluralité de zones comprenant chacune des compositions à base de mélanges de cristaux liquides différents, réagissant chacune à des températures différentes, de manière à "s'allumer" successivement en fonction de la quantité de radiation infrarouge reçue.

Avantageusement, le dispositif selon l'invention est configuré sous forme d'une casquette (avec ou sans fond) ou d'un chapeau (de type comportant un rebord relativement rigide entourant l'extrémité du chapeau opposée à son fond. La portion de la casquette ou du chapeau recouvrant la tête en totalité (ou en partie dans le cas d'une casquette sans fond) fait donc office de moyen de fixation du dispositif de détection de sorte que ce dernier soit positionné correctement relativement au rayonnement solaire.

De manière classique, la casquette peut comprendre des moyens, notamment sous forme d'une ou plusieurs bandes élastiques, ou sous forme d'un système d'accrochage à position variable, permettant d'assurer une bonne tenue de la casquette sur la tête.

Dans le cas d'une casquette, les moyens de détection peuvent être disposés sur une première visière, destinée à être positionnée en regard de la nuque de l'individu.

Avantageusement, la casquette comprend une seconde visière, située à l'opposé de la première. La seconde visière utilisée classiquement pour protéger le visage, et en particulier les yeux, du soleil, sert en outre de repère pour assurer un positionnement adéquat de la première visière, à savoir celle sur laquelle sont fixés les capteurs.

Selon un autre aspect, l'invention concerne une casquette comprenant :
i) une portion destinée à sa fixation sur la tête d'un individu ;
ii) une première visière destinée à se positionner sensiblement en regard de la nuque du sujet, la première visière étant équipée de moyens de détection, comprenant au moins un capteur sensible à au moins un rayonnement solaire, notamment UV, et de moyens couplés aux moyens de détection pour générer un signal représentatif de la quantité de rayonnement reçue par les moyens de détection ; et
iii) une seconde visière située à l'opposé de la première.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 représente une casquette constituant un mode de réalisation préférentiel du dispositif selon l'invention ;
- la figure 2 illustre un mode de réalisation du système de détection/affichage équipant la casquette de la figure 1 ;
- la figure 3 illustre la position du dispositif de détection/affichage de la figure 2 pour deux positions du sujet dont une est autre que debout ; et
- la figure 4 illustre une variante de la figure 1.

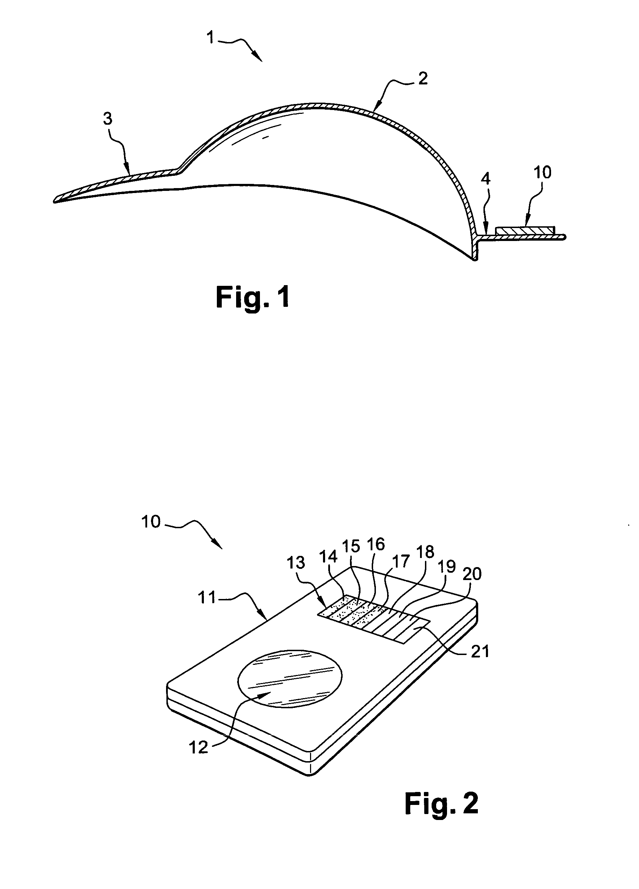

La casquette 1 illustrée à la figure 1 comprend une portion en tissu 2 destinée à recouvrir de manière légèrement serrante le crâne d'un sujet, en particulier d'un enfant.

La casquette 1 comprend une visière 3 (en carton épais ou plastique recouvert de tissu) destinée à se positionner classiquement en regard du visage du sujet.

A l'opposé de la visière 3, la casquette 1 comprend une autre visière 4 sur la surface supérieure de laquelle est disposée une unité de détection/ affichage 10 qui va être décrite de manière détaillée en référence à la figure 2. La forme du dispositif de détection/affichage 10 est similaire à celle d'une carte de crédit.

Le dispositif 10 est fixé à la visière 4 de manière irréversible, ou mieux encore, de manière réversible. Dans ce dernier cas, la fixation est assurée au moyen de fixations de type VELCRO© . Ainsi, préalablement au nettoyage de la casquette, le dispositif 10 peut être enlevé.

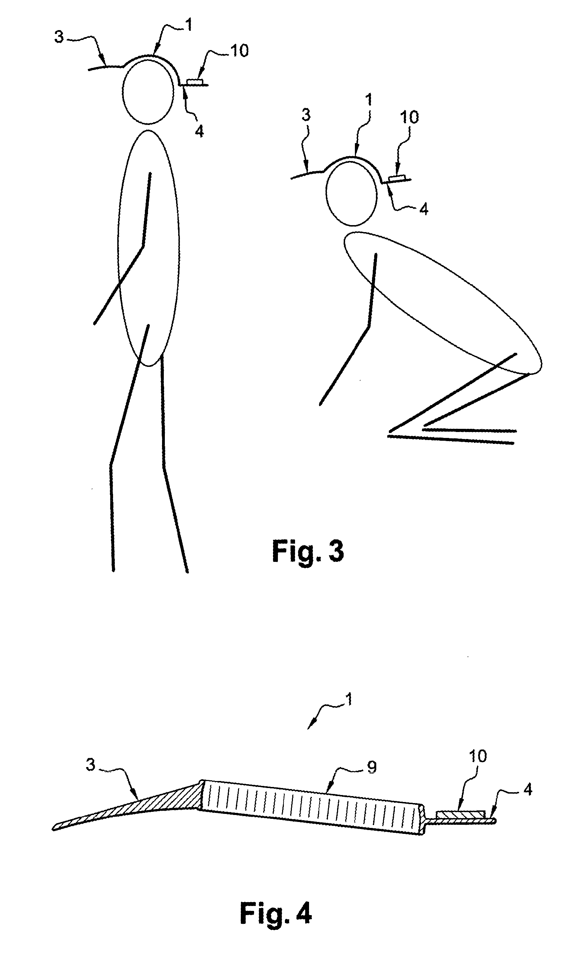

La visière 4 est réalisée en un matériau identique à celui de la visière 3 et est destinée à être positionnée en regard de la nuque du sujet. Sa forme et ses dimensions sont choisies de manière à limiter la gêne qu'elle pourrait occasionner, en particulier lorsque le sujet est dans une position autre que debout, comme représenté à la figure 3 à laquelle il sera fait référence ultérieurement.

Le dispositif de détection/affichage 10 représenté à la figure 2 comporte un support en plastique 11 à la forme et aux dimensions d'une carte de crédit.

Le support 11 comprend deux feuilles en plastique superposées et entre lesquelles sont disposés les moyens de détection et d'affichage.

La feuille supérieure comprend une première fenêtre en regard de laquelle se trouve la partie active d'une photocellule 12 et une seconde fenêtre en regard de laquelle se trouve une unité d'affichage 13.

La cellule photovoltaïque 12 est reliée par un couplage électronique approprié à l'unité d'affichage 13.

L'unité d'affichage 13 comporte une pluralité de zones 14-21 aptes à changer de couleur successivement en fonction de la température à laquelle elles sont portées, via une ou plusieurs résistances traversée(s) par le courant généré par la cellule photovoltaïque 12.

Les premières zones 14-18 de la rangée contiennent un composé à cristaux liquides thermochromes qui devient vert lorsqu'une température environ égale à 35 °C est atteinte. Les suivantes 19-20 contiennent un autre composé à cristaux liquides thermochromes qui devient orange lorsqu'une température environ égale à 45 °C est atteinte. La dernière zone 21 de la rangée contient encore un autre composé thermochrome qui devient rouge lorsqu'une température d'environ 55 °C est atteinte. Le passage au rouge de la dernière zone 21 indique au sujet qu'il est temps de réappliquer du produit de protection.

Des compositions à base de tels mélanges de cristaux liquides thermochromes sont décrites en particulier dans le brevet US 4 863 283 auquel il a été fait référence précédemment.

Comme il apparaît à la figure 3, que le sujet soit en position debout ou encore accroupie ou à genoux, la surface de la visière arrière 4 de la casquette 1 reste sensiblement horizontale et parallèle au sol. De ce fait, le capteur de l'unité de détection/affichage 10 est toujours orienté de façon sensiblement perpendiculaire au rayonnement solaire.

Ainsi, et à l'opposé des systèmes classiques délivrant généralement une information "minorée" quant à la quantité de rayonnement effectivement reçue par le sujet, le dispositif selon l'invention donne une information correspondant sensiblement à l'exposition maximale à laquelle le sujet a pu être exposé.

Selon la variante de la figure 3, la casquette 1 est sans fond et comprend essentiellement une bande de serrage 9, notamment en plastique, destinée à entourer la tête. Le serre-tête 9 est équipé des deux visières 3 et 4 discutées en référence au mode de réalisation précédent.

Dans la description détaillée qui précède, il a été fait référence à des modes de réalisation préférés de l'invention. Il est évident que des variantes peuvent y être apportées sans s'écarter de l'esprit de l'invention telle que revendiquée ci-après.

## Revendications

1. Dispositif (1) pour prévenir les risques d'une surexposition aux rayonnements néfastes du soleil :
i) des moyens de détection (12), comprenant au moins un capteur sensible à au moins un rayonnement solaire, notamment UV ;
ii) des moyens (13) couplés aux moyens de détection pour générer un signal représentatif de la quantité de rayonnement reçue par les moyens de détection (12), et
iii) des moyens (2, 9) de fixation du dispositif sur la tête d'un sujet, de sorte que le(s) capteur(s) puisse(nt) être orienté(s) de façon sensiblement perpendiculaire à la verticale du sujet lorsque celui-ci est debout,
le dispositif étant configuré sous forme d'une casquette, les moyens de détection (12) étant disposés sur une première visière (4), destinée à être positionnée en regard de la nuque de l'individu, la casquette comprend une seconde visière (3), située à l'opposé de la première.

2. Dispositif (1) selon la revendication 1 **caractérisé en ce que** le signal est visuel et/ou audible.

3. Dispositif (1) selon la revendication 1 ou 2 **caractérisé en ce que** les moyens (13) pour générer ledit signal sont distincts des moyens de détection (12).

4. Dispositif (1) selon la revendication 3 **caractérisé en ce que** les moyens de détection (12) comprennent une photocellule, notamment une cellule photovoltaïque.

5. Dispositif (1) selon la revendication 1 ou 2 **caractérisé en ce que** les moyens de détection (12) génèrent le signal représentatif de la quantité de rayonnement reçue.

6. Dispositif (1) selon l'une quelconque des revendications 1 à 5 **caractérisé en ce que** les moyens pour générer ledit signal (13) comprennent au moins une zone (14-21) comprenant une composition contenant au moins un composé photochrome ou thermochrome, notamment à base de cristaux liquides ou de composés fluorescents.

7. Casquette (1) comprenant :
i) une portion (2, 9) destinée à sa fixation sur la tête d'un sujet ;
ii) une première visière (4) destinée à se positionner du côté de la nuque du sujet, la première visière (4) étant équipée de moyens de détection (12), comprenant au moins un capteur sensible à au moins un rayonnement solaire, notamment UV, et de moyens (13) couplés aux moyens de détection pour générer un signal représentatif de la quantité de rayonnement reçue par les moyens de détection ;
iii) une seconde visière (3) située sensiblement à l'opposé de la première.
